(19) Europäisches Patentamt
European Patent Office
Office européen des brevets

(11) **EP 4 172 275 B1**

(12) **EUROPEAN PATENT SPECIFICATION**

(45) Date of publication and mention
of the grant of the patent:
**07.02.2024 Bulletin 2024/06**

(21) Application number: **21791117.1**

(22) Date of filing: **15.09.2021**

(51) International Patent Classification (IPC):
*C09C 1/00* (2006.01)   *C09C 1/42* (2006.01)
*A61K 8/02* (2006.01)   *A61K 8/24* (2006.01)
*A61K 8/25* (2006.01)   *A61Q 1/08* (2006.01)

(52) Cooperative Patent Classification (CPC):
**C09C 1/42; A61K 8/0279; A61K 8/24; A61K 8/25;
A61Q 1/08; C09C 1/00;** A61K 2800/412;
A61K 2800/621; C01P 2004/03; C01P 2004/51;
C01P 2004/61; C01P 2004/62; C01P 2004/64

(86) International application number:
**PCT/US2021/050382**

(87) International publication number:
**WO 2022/060779 (24.03.2022 Gazette 2022/12)**

(54) **TIO2-FREE PIGMENT**

TIO2-FREIES PIGMENT

PIGMENT EXEMPT DE TIO2

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.09.2020 US 202063078974 P**

(43) Date of publication of application:
**03.05.2023 Bulletin 2023/18**

(73) Proprietor: **Sun Chemical Corporation
Parsippany, NJ 07054 (US)**

(72) Inventors:
• **DOLL, Jonathan
Cincinnati, OH 45232 (US)**
• **BENNETT, Jamie
Cincinnati, OH 45232 (US)**

(74) Representative: **Carpmaels & Ransford LLP
One Southampton Row
London WC1B 5HA (GB)**

(56) References cited:
**WO-A1-2017/127362**

• **FARRAH S R ET AL: "Use of modified
diatomaceous earth for removal and recovery of
viruses in water", APPLIED AND
ENVIRONMENTAL MICROBIOLOGY, AMERICAN
SOCIETY FOR MICROBIOLOGY, US, vol. 57, no.
9, 1 September 1991 (1991-09-01), pages
2502-2506, XP008157350, ISSN: 0099-2240**
• **DONG XIONGBO ET AL: "A novel rutile
TiO2/AlPO4 core-shell pigment with substantially
suppressed photoactivity and enhanced
dispersion stability", POWDER TECHNOLOGY,
ELSEVIER, BASEL (CH), vol. 366, 6 March 2020
(2020-03-06), pages 537-545, XP086117958, ISSN:
0032-5910, DOI: 10.1016/J.POWTEC.2020.03.002
[retrieved on 2020-03-06]**
• **SZEWCZYK ADRIAN ET AL:
"Microwave-Assisted Fabrication of Mesoporous
Silica-Calcium Phosphate Composites for Dental
Application", POLYMERS, vol. 13, no. 1, 25
December 2020 (2020-12-25), page 53,
XP055875509, CH ISSN: 2073-4360, DOI:
10.3390/polym13010053**

**Description**

**BACKGROUND**

The interaction of cosmetic formulations with the skin and facial oils or sweat has a large impact on the formulation's wear properties. Facial oils, such as sebum, can wet the pigments within a cosmetic formulation, causing them to migrate on the skin. Additionally, when a pigment becomes wetted by sebum it can undergo a color shift resulting in an unnatural appearance. These pitfalls manifest as an appearance of "cakiness" which more readily exposes fine lines and wrinkles. Regardless of how it manifests, the cosmetic must be reapplied, and consumers of cosmetics consider regular reapplication as a negative.

One way to mitigate the effect of migration is to use colourless filler particles with a high surface area that sacrificially absorb oil before the cosmetic becomes fully wetted. These high surface area fillers are scavengers for facial oil, absorbing it before it interacts with the rest of the formulation, and increasing the amount of time a cosmetic can be worn prior to the onset of migration.

Another way to mitigate the effect of migration is to include particles that obscure or blur fine lines and wrinkles. Such a phenomenon is called soft focus. In the case where a cosmetic migrates to a facial wrinkle, the pigment providing soft focus would obscure the wrinkle, making it less obvious, and decreasing the need to reapply the make-up.

Historically, cosmetic materials either address the oil absorption or the soft focus, but none have addressed both issues simultaneously. Traditionally, these cosmetic materials comprised substances including, but not limited to, talc, titanium dioxide, zinc oxide, and/or microplastics, ingredients which have recently been perceived as harmful by consumers and/or regulatory agencies. More recently, the public's concern over these ingredients, has led to new regulations worldwide with respect to their use and labelling for cosmetics. For this reason, it would be beneficial to develop a product using materials that are generally recognized as safe (GRAS materials).

Traditional strategies to improve the wear of a cosmetic formulation have focused on the binder, utilizing highly hydrophobic polymers, silicones, and pigment surface treatments to create a film over the cosmetic that prevents smudging. This approach protects against external environmental factors like sweat and tears that originate from an area apart from where the makeup is applied.

Soft focus pigments are available, however they contain ingredients that are considered by the public to be harmful to human health or the environment. These ingredients include titanium dioxide, talc, boron, and microplastics. Prior art soft focus pigments which are not as highly oil absorbent may or may not experiences consequences as a result BUT our pigment acts as a two in one - absorbing filler to prevent the soil from interacting with other portions of the cosmetic as well as a soft focus effect. These oils can cause a cosmetic formulation to migrate, resulting in the need to reapply the formulation after a short period of time.

This current composition addresses these issues by forming a layered pigment system comprising a porous or non-porous substrate with a porous shell. One commercial embodiment may comprise a diatomaceous earth substrate with a hydroxyapatite shell to form a functional pigment system with desirable properties.

The combination of these two minerals into a single particle has been found to increase particle surface area, which increases oil absorption when applied to the skin and desirable soft focus properties due to the high porosity of the powders.

Farrah et al., Applied and Environmental Microbiology, vol. 57, no. 9, 1991, pp. 2502-2506 relates to the use of modified diatomaceous earth for the removal and recovery of viruses in water. The diatomaceous earth was modified by in situ precipitation of metallic hydroxides.

WO 2017/127362 A1 discloses a soft-focus composition comprising particles, wherein the particles comprise a substrate with a metal oxide coating that covers from about 50% to about 75% of the surface area of the substrate.

Dong et al., Powder Technology, vol. 366, 2020, pp. 537-545 discloses a rutile $T_1O_2/AlPO_4$ core-shell pigment with substantially suppressed photoactivity and enhanced dispersion stability, which is constructed via a wet chemical deposition method.

**DESCRIPTION OF FIGURES**

   **Figure 1:** SEM image of Comparative Example 7

   **Figure 2:** SEM imaging of Example 1

## SUMMARY

The current composition is directed to a layered pigment system that incorporates an outer layer which is a porous mineral shell that is coated onto an inner substrate particle, that may or may not be porous, to form a layered pigment system, wherein the porous mineral shell comprises a calcium phosphate mineral. Both the inner substrate and outer layer generally have a naturally porous microstructure, which may produce soft-focus properties as well as increased oil absorption, when applied to a surface. This may reduce pigment migration and saturation of a cosmetic formulation once applied to the skin.

The porous mineral shell comprises a calcium phosphate mineral, for example hydroxyapatite (HA), and the inner particle may be diatomaceous earth. Both diatomaceous earth (DE) and hydroxyapatite (HA) are porous, and the combination of the two materials results in a functional layered pigment system with a high degree of surface area. The high surface area leads to excellent absorption of oils when compared to other commonly used fillers. Moreover, the low refractive index of HA and the substrate and the porous, bilayer structure resulting in many different material interfaces (*e.g.* air-HA, air-substrate, HA-substrate), which leads to strong forward scattering and diffusion of light, resulting in excellent soft focus. The combination of both high oil absorption and soft focus has not been demonstrated before, and this technology allows formulators to capitalize on both properties while using a single ingredient.

Hydroxyapatite (HA) is a mineral of hydrated calcium phosphate with the following chemical formula: $Ca_{10}(PO_4)_6(OH)_2$. It is the main mineral that makes up tooth enamel and makes up to 70% the mass of bone in mammals. Hydroxyapatite can be easily precipitated from solution to give a porous mineral coating. Such coatings are used in fertilizer and in organ transplants as they are highly biocompatible and promote the growth of cellular material.

Diatomaceous earth (DE) is a porous mineral made from fossilized algae that is used in a number of applications, including cosmetics, agriculture and food. On a molecular level, DE is made of silicon dioxide, although other minerals like $Ca^{2+}$ and $Mg^{2+}$ may also be present. The porosity in the mineral results from the structure of the original algae organism prior to fossilization.

Combining materials such as diatomaceous earth (DE) and hydroxyapatite (HA) into a layered pigment system provides advantages over the prior art in that both substances are generally perceived as safe. The combination of the generally safe ingredients with the technical advantages, produces a compelling value proposition for cosmetic manufactures and provides a good incentive to commercialization.

## DETAILED DESCRIPTION

The layered pigment system described in this application comprises a layered pigment particle having a porous or non-porous mineral substrate and a mineral shell. Note that "porous mineral substrate core," "porous mineral substrate," and "porous substrate" may be used interchangeably. The high porosity and multilayer structure of the pigment causes the pigment to have a high oil absorption, which can prolong the longevity of a cosmetic formulation. The pigment structure also leads to excellent soft focus properties when it is used in a cosmetic formulation. This layered pigment system may further comprise other additives which are discussed below.

Without being bound to theory, it is thought that the bilayer structure of porous materials allows for more efficient oil absorption than either a blend of the materials or an equal volume amount of the individual materials. Moreover, this layered combination of porous materials creates a structure with high surface area and a number of interfaces, which lead to efficient blurring and soft focus.

In one embodiment, the layered pigment system comprises an inner porous substrate and an outer shell, wherein the term "porous substrate" implies that it has a high amount of surface area, relative to a non-porous substrate of equal size. While the pore morphology is not important, the pores may be spherical, cylindrical, amorphous or lamellar without reducing the scope of the invention.

In one embodiment, the porous substrate is a mineral selected from: silica, mica, perlite, diatomaceous earth, kaolin, kaolinite, sericite, clay, talc, diatomite, zeolite, and mixtures thereof.

In one embodiment, the porous substrate is a high surface area particle. In the case where the substrate is a particle, the substrate can have a shape that defines its boundaries. The shape of the substrate does not limit the scope of the invention, and the substrate can be any shape known to those skilled in the art. Exemplary substrate shapes include, irregular, spherical, platelet, acicular, wire, and mixtures thereof. The substrate has a particle size distribution that is defined by its median particle size, d50, as measured by light scattering. The d50 of the substrate is preferably in the range of 2-100 $\mu$m. In one embodiment, the d50 of the substrate may be in the range of 0.5-35 $\mu$m.

The substrate is coated with one or more layers of a porous mineral shell, wherein the porous mineral shell comprises a calcium phosphate mineral. A porous mineral shell is defined as the mineral shell having a high surface area with the pores having any morphology. In the case of the porous mineral shell, it may be present on the surface of the porous mineral substrate or it may penetrate into the pores of the porous mineral substrate. The porous mineral shell is generally present on the surface of the porous substrate in a range of 1-100% by weight with respect to the porous substrate. The porous mineral shell may fully or partially encompass the porous substrate without limiting the scope of the invention.

In one embodiment the porous mineral shell may be crystalline or amorphous without limiting the scope of the invention. Porous mineral shells may include, but are not limited to, one or more minerals from the following group, including calcium phosphate, monocalcium phosphate, monocalcium phosphate monohydrate, dicalcium phosphate, dicalcium phosphate dihydrate, dicalcium phosphate monohydrate, tricalcium phosphate, tetracalcium phosphate, octacalcium phosphate, dicalcium diphosphate, calcium triphosphate, calcium hydroxy phosphate, monetite, brushite, apatite, hydroxyapatite, and mixtures thereof.

In one embodiment, the layered pigment system may be optionally blended or treated with one or more additives. These additives may have different functions, such as improving dispersibility, improving feel, improving the hydrophobicity, and improving oil absorption. Additionally, the additives may be used to modify the surface charge characteristics of the layered pigment system to be cationic, anionic, neutral or uncharged.

In one embodiment, the layered pigment system may also contain ingredients that may be passively released at a later time in an application. This is possible due to the porous structure of the particle and creating a large amount of free volume per particle. Examples of typical additives include, but are not limited to, one or more from the following: methicone, dimethicone, trifluoropropyl dimethicone, lecithin, egg lecithin, vegetable lecithin, hydrogenated lecithin, galactose arabinan sugar, starch, alginic acid, sodium alginate, potassium alginate, chitosan, magnesium myristate, aluminum myristate, zinc myristate, sodium glycerophosphate, alanine, arginine, asparagine, aspartic acid, sodium aspartate, cysteine, glutamine, glutamic acid, sodium glutamate, glycine, proline, histidine, isoleucine, leucine, lysine, methionine, phenylalanine, serine, threonine, tryptophan, tyrosine, valine, taurine, citruline, ornithine, theanine, dipeptides, tripeptides, polypeptides of more than three amino acids, proteins, enzymes, betaine, carnitine, carnosine, hydroxytryptophan, cysteine, hydroxyproline, N-acetyl cystine, S-adenosyl methionine, tyramine, $\gamma$-aminobutyric acid, serotonin, dopamine, 2-aminohepanoic acid, 2-aminoisobutyric acid, 3-aminoisobutyric acid, 2-aminopimelic acid, 2,4-diaminobutyric acid, desmosine, 2,2'-diaminopimelic acid, 2,3-diaminopropionic acid, N-ethylgllycine, selenomethionine, allo-isoleucine, N-methylglycine, N-methylisoleucine, 6-N-methyllysine, N-methylvaline, norvaline, norleucine, pyrrolysine, formylmethionine, $\beta$-alanine, $\delta$-aminolevulinic acid, 4-aminobenzoic acid, dehydroalanine, cystathionine, lanthionine, djenkolic acid, diaminpimelic acid, isovaline, lauroyl lysine, glutamate-cysteine-arginine peptides, sodium myristoyl sarcosinate, disodium steroyl glutamate, fatty acids, lipids, stearic acid, sodium stearate, oleic acid, sodium oleate, palmitic acid, sodium palmitate, myristic acid, elaidic acid, sodium elaidate, sodium myristate, lauric acid, sodium laurate, arachidic acid, sodium arachidate, erucic acid, sodium erucate, palmitoleic acid, sodium palmitoleate, linoleic acid, sodium linoleate, triethoxyoctyl silane, trimethoxyoctyl silane, triethoxydecyl silane, trimethoxydecyl silane, triethoxydodecyl silane, trimethoxydodecyl silane, triethoxytetradecyl silane, trimethoxytetradecyl silane triethoxyhexadecyl silane, trimethoxyhexadecyl silane, triethoxyoctadecyl silane, trimethoxyoctadecyl silane, PEG-8 triethoxysilane, jojoba wax, polyethylene wax, carnauba wax, unreacted fluorinated compounds, isopropyltitanium triisostearate, perfluoro alkyl phosphates, triethoxylcapryl silane, stearoyl glutamic acid, perfluoroocctyl triethoxysilane, silica, aloe, and mixtures and combinations thereof.

The layered pigment system comprising a porous mineral substrate and a porous mineral shell may be used in cosmetic formulations to improve the long wear properties of the cosmetic. In one embodiment, the layered pigment system may be incorporated into any type of personal care or cosmetic formulation at an optimized loading that improves the wear properties. In another embodiment, the layered pigment system may be incorporated into any type of personal care or cosmetic formulation at an optimized loading that shows a maximum in the soft focus properties of a formulation. In one embodiment, the layered pigment system may be an ingredient that provides a passive or an active effect to a cosmetic. In another embodiment, the layered pigment system may act as a filler or binder in a cosmetic formulation. In one embodiment the optimized loading of the layered pigment system may be in the range of 0.1% - 90.0% by weight, with regards to the total weight of the personal care or cosmetic formulation, depending on the type of cosmetic formulation.

In one embodiment, the layered pigment system may be incorporated into any type of personal care formulation such as acne treatments, face creams, skin gels, hand creams, body lotions, moisturizers, water in oil formulation, oil in water formulations, cellulite treatments, body splashes, shampoos, conditioners, styling products, hair sprays, setting lotions, primers, mousses, gels, pomades, waxes, dry shampoos, serums, oils, hair color, root touch up products, scalp treatments, deodorants, antiperspirants, sun screens, tanning lotions, skin lighteners, lip balms, anti-aging creams, eye serums, body oils, make up removers, shaving creams, shaving gels, and eye creams. In the case where the layered pigment is incorporated into a personal care formulation, the layered pigment may be loaded with one or more active or passive ingredients for immediate or prolonged release without limiting the scope of the invention.

In another embodiment, the layered pigment system may be incorporated into any cosmetic formulation such as foundations, pressed powders, loose powders, bronzers, concealers, BB/CC creams, tinted moisturizers, liquid foundations, eye shadows, eye liners, lipsticks, lip glosses, blushes, rouges, facial powders, and nail polishes.

In one embodiment, the layered pigment system may be incorporated into an ink or coating. The ink or coating created may have an aesthetically desirable light diffusion effect. The soft focus effect of the coating or ink can be one or more of a variety of effects, ranging from, but not limited to, opacification, matting, haziness, blemish hiding, and extending. Examples of inks and coatings include but are not limited to automotive coatings, protective clear coatings, interior architectural coatings, exterior architectural coatings, powder coatings, industrial coating, anti-corrosion coating, gravure inks, flexographic inks, paste inks, energy curing (UV or EB) inks, etc. Additionally, the layered pigment may be used in combination with other effect pigment or organic pigments in all ratios without limiting the scope of the invention.

The content of the layered pigment system in the coating or ink composition may be set preferably in the range of 0.1% to 50% by weight with respect to the other components of the coating system. The content of the layered pigment system may also be set in the range of 1% to 20% by weight, with respect to the other components of the coating system.

In one embodiment, the layered pigment system may be incorporated into a plastic part. In this embodiment, the plastic according to the present invention may be obtained by incorporation into the layered pigment system and into a plastic material by compounding the layered pigment system with a plastic at temperatures above the glass transition temperature of the plastic. Suitable methods for incorporating the layered pigment system may include, but are not limited to, blow molding, extrusion or other techniques used to make plastic films or articles known to those skilled in the art. In one embodiment, the layered pigment system may be incorporated into a plastic at a loading in the range of 0.01% - 20% with respect to the total weight of the formulation.

In the case where the layered pigment system is incorporated into a plastic, any suitable plastic may be used including, but not limited to, polypropylene, polyethylene, polyester, polyurethane, polyacrylate, polyolefin, epoxy, polyamide, poly(vinyl chloride), and poly(vinylidene fluoride), as well as any acrylic, alkyd, fluoropolymers, and blends thereof. In the case where the layered pigment system is incorporated into a plastic it can be used in conjunction with one or more additional light diffusing or colored pigments without limiting the scope of the invention.

## EXAMPLES

The invention is further described by the following non-limiting examples which further illustrate the invention, and are not intended, nor should they be interpreted to, limit the scope of the invention.

Example 1:

In a 2 L oil jacketed reactor, 26.4 g of diatomaceous earth (Imerys Imercare 18D) was added with 600 g of D.I. water. The substrate was stirred in water at 350 RPM and heated to 70 °C. 83.2 g of calcium acetate hydrate and 102.6 g of ethylenediaminetetraacetic acid and 65 g water was added to the reactor and stirred for 30 min. 37 g of 87% phosphoric acid was added. 50% sodium hydroxide was metered into the reactor until pH reached 10.0. Once all reagents were added, the slurry was stirred for 30 min. The slurry was filtered and washed with water. The aqueous paste was then dried at 60°C, resulting in a white powder comprised of about 51% hydroxyapatite.

Example 2:

In a 1 L oil jacketed reactor, 30 g of Imerys Imercare 18D was added with 400 g of D.I. water. The substrate was stirred in water at 350 RPM and heated to 55 °C. 47.3 g of calcium acetate hydrate and 58.3 g of ethylenediaminetetraacetic acid was added to the reactor and stirred for 30 min. 20.5 g of 87% phosphoric acid was added. 10% sodium hydroxide was metered into the reactor until pH reached 10.5. Once all reagents were added, the slurry was stirred for 30 min. The slurry was filtered and washed with water. The aqueous paste was then dried at 60°C, resulting in a white powder comprised of about 23% hydroxyapatite.

Example 3:

In a 2 L oil jacketed reactor, 20 g of Imerys Imercare 18D was added with 800 g of D.I. water. The substrate was stirred in water at 350 RPM and heated to 75°C. 220 g of calcium acetate hydrate and 270.9 g of ethylenediaminetetraacetic acid was added and stirred for 30 min. 95.4 g of 87% phosphoric acid was added. 50% sodium hydroxide was metered into the reactor until pH reached 9.5. Once all reagents were added, the slurry was stirred 30 min. The reaction contents were removed from the reactor, filtered, and washed. The aqueous paste was then dried at 60°C, resulting in a white powder comprised of about 77% hydroxyapatite.

Example 4:

In a 1L oil jacketed reactor, 10.3g of kaolin (Imery's Imercare 02K) was added with 270g of D.I. water, 78.8g calcium acetate hydrate, and 48.6g ethylenediaminetetraacetic acid. Materials were stirred at 350 RPM and heated to 75°C. Once at temperature, 35g 86% phosphoric acid was added. 180g of 40% sodium hydroxide solution was metered into the reactor slowly. Reaction contents were removed from the reactor, filtered, and washed. The aqueous paste was then dried at 60°C resulting in a white powder comprising ~60% hydroxyapatite.

Example 5:

In a 2 L oil jacketed reactor, 30 g of diatomaceous earth (Imerys Imercare 03D) was added with 600 g of D.I. water. The substrate was stirred in water at 350 RPM and heated to 60 °C. 94.6 g of calcium acetate hydrate and 116.6 g of ethylenediaminetetraacetic acid was added and stirred for 30 min. 42 g of 87% phosphoric acid was added. 50% sodium hydroxide was metered into the reactor until pH reached 10.0. Once all reagents were added, the slurry was stirred 30 min. The reaction contents were removed from the reactor, filtered, and washed. The aqueous paste was then dried at 60°C, resulting in a white powder comprised of about 77% hydroxyapatite.

Example 6:

In a 1L oil jacketed reactor, 13.4g of mica (C86-6105 Sun Chemical) was added with 270g of D.I. water, 78.8g calcium acetate hydrate, and 48.6g ethylenediaminetetraacetic acid. Materials were stirred at 350 RPM and heated to 75°C. Once at temperature, 35g 86% phosphoric acid was added. 180g of 40% sodium hydroxide solution was metered into the reactor slowly. Reaction contents were removed from the reactor, filtered, and washed. The aqueous paste was then dried at 60°C resulting in a white powder comprising ~60% hydroxyapatite.

Note: All Comparative Examples lack calcium phosphate mineral, particularly hydroxyapatite.

Comparative Example 7: Imerys Imercare 18D - Diatomaceous earth (no hydroxyapatite)

Comparative Example 8: Barretts Minerals MP1538USP - talc

Comparative Example 9: Imerys Imercare 02K - Kaolin (no hydroxyapatite)

Comparative Example 10: Sun Chemical C86-6105 - mica (no hydroxyapatite)

Comparative Example 11: Sun Chemical C47051 - $TiO_2$ (white pigment used for soft focus)

Comparative Example 12: Sun Chemical SpectraFlex Illusion C88-0103 - 25% $TiO_2$ on talc (white pigment used for soft focus)

Comparative Example 13: Saint-Gobain Ceramics & Plastics Tres BN PUHP3002 - hexagonal boron nitride (white pigment used for soft focus)

Comparative Example 14: 25% TiO2 on Imercare 18D Diatomaceous earth (white pigment used for soft focus)

Particle Size

The particle size of Examples 1-14 was measured using a Cilas 1064L. Sample was prepared in water at 5% and sonicated for 5min. The median particle size, d50, is reported in Table 1.

Oil Absorption

Castor oil was slowly added to 1 gram of pigments in Examples 1-13 until the pigment became wetted. A spatula was used to work the oil into the dry pigment. The amount of oil required to wet 1g of pigments was noted and is reported in Table 1. Reported values are an average of 3 measurements.

Table 1: Particle size (d50), oil absorption and surface area of Examples 1-14

| Sample | d50 ($\mu$m) | Oil absorption ($g_{oil}/g_{pigment}$) | Specific Surface area ($m^2/g$) |
|---|---|---|---|
| Inv. Example 1 | 16.42 | 1.84 | 41.4 |
| Inv. Example 2 | 14.67 | 1.37 | 18.7 |
| Inv. Example 3 | 16.86 | 1.90 | 37.3 |

(continued)

| Sample | d50 (μm) | Oil absorption ($g_{oil}/g_{pigment}$) | Specific Surface area (m²/g) |
|---|---|---|---|
| Inv. Example 4 | 10.65 | 1.03 | 46.8 |
| Inv. Example 5 | 14.51 | 1.51 | 25.6 |
| Inv. Example 6 | 14.57 | 1.42 | 47.8 |
| Comp. Example 7 | 15.98 | 0.83 | 5.5 |
| Comp. Example 8 | 8.19 | 0.58 | 9.4 |
| Comp. Example 9 | 13.19 | 0.73 | 7.7 |
| Comp. Example 10 | 13.65 | 0.54 | 5.4 |
| Comp. Example 11 | 0.33 | 0.31 | 8.9 |
| Comp. Example 12 | 5.05 | 0.79 | 33.8 |
| Comp. Example 13 | 9.23 | 1.11 | 9.5 |
| Comp. Example 14 | 15.1 | 0.76 | 68.2 |

The data in Table 1 shows that the inventive Examples 1-6 have high oil absorption values of >1.0 $g_{oil}/g_{pigment}$, indicating that they will be longer lasting when applied in a cosmetic film to the skin than Comparative Examples 7-12 and 14, which have oil absorption values of <1.0 $g_{oil}/g_{pigment}$. Although example 13 has high oil absorption, its composition is not considered to be environmentally friendly or safe in the consumer's perception.

Surface Area

The surface area was measured by $N_2$ adsorption using a Nova 2000e. The results of the surface area measurements are reported in Table 1. The results show that the surface area of the inventive examples is much higher than the comparative examples.

SEM Analysis

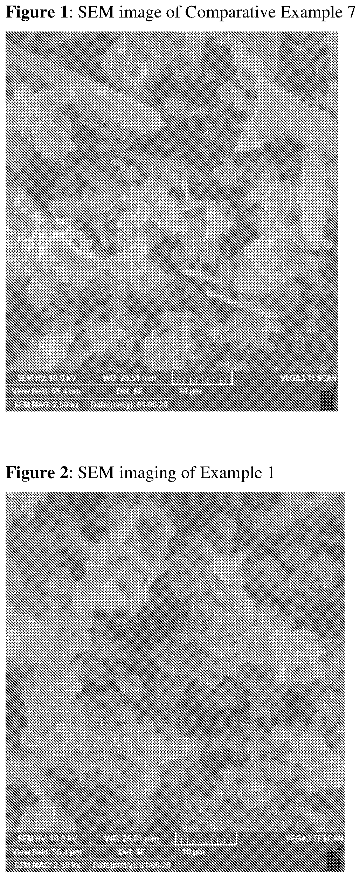

The SEM micrograph of Examples 1 and Comparative Example 7 were measured using a Vega3 Tescan. The SEM images are reported in Figure 1 and 2. Figure 1 shows an SEM image of Comparative Example 7, which is diatomaceous earth without a hydroxyapatite layer. In Figure 1, you can see the small particles which have a geometrical shape and smooth surfaces. Figure 1 can be compared in contrast to Figure 2 which has a more textured appearance, indicative of its higher surface area. The textured surface results from the deposition of the porous hydroxyapatite layer.

Soft Focus Measurements

The soft focus of the pigments is an optical phenomenon leading to blurring and obscuring of fine lines and wrinkles. The soft focus may be quantified by a method that incorporates both the reflective and transmissive properties of a pigment film.

The reflective portion of the soft focus is known as $SFF_R$, and it is defined in Equation 1 as:

$$SFF_R = L*75/L*15 \ (1)$$

where L*15 and L*75 are the L* brightness values as measured in a multiangle spectrophotometer using a 45° incident beam and measuring the reflected beam at an aspecular reflectance angle of 15° and 75°. A good soft focus effect is observed, when the $SFF_R$ is between 0.4-0.7.

The transmissive portion of the soft focus ($SFF_T$) is defined by Equation 2 as:

$$SFF_T = T_{TOT} \ T_{DIF} \ (2)$$

where $T_{TOT}$ and $T_{DIF}$ are the total and diffuse transmittance of a film as measured by spectrophotometer with the diffuse sphere configuration. A good soft focus effect is observed, when the SFFr is above 0.50.

To measure the soft focus, the examples were dispersed into a solvent-borne cellulose acetate butyrate paint base at 10% (w/w) loading. The paints were drawn down using a 1.5 mil Bird applicator onto a black and white test card (Byk Chart 2811). The multiangle color data was measured over the black portion of the card using a BYK mac i multiangle spectrophotometer. An additional drawdown was made on a transparent Mylar sheet using a 1.5 mil Bird applicator. The direct and total transmittance between 400-700nm were measured using an X-Rite Color i7 spectrophotometer. The SFFr and $SFF_R$ are reported in Table 2.

Table 2: Soft Focus measurements of Examples 1-13

| Sample | $SFF_R$ | $SFF_T$ |
|---|---|---|
| Inv. Example 1 | 0.41 | 0.75 |
| Inv. Example 3 | 0.70 | 0.71 |
| Inv. Example 4 | 0.48 | 0.59 |
| Inv. Example 6 | 0.47 | 0.52 |
| Comp. Example 7 | 0.32 | 0.50 |
| Comp. Example 9 | 0.16 | 0.32 |
| Comp. Example 11 | 0.96 | 0.09 |
| Comp. Example 12 | 0.57 | 0.39 |
| Comp. Example 13 | 0.81 | 0.26 |
| Comp. Example 14 | 0.37 | 0.72 |

The data shows that Comparative Example 12, which is comprised of $TiO_2$ coated onto talc has a soft focus effect, with both $SFF_R$ and $SFF_T$ falling into the prescribed ranges. Comparative Example 13, which is a born nitride powder marketed for soft focus appears opaque and the measured soft focus falls out of the range at this loading. In contrast, Example 1, shows a strong soft focus effect at this loading, with an $SFF_T$ that is the highest of all the samples and a visible haze effect that is indicative of soft focus.

Table 3: Recipe of a CC cream containing the examples of the invention

| φ | Ingredients | INCI | % |
|---|---|---|---|
| | Water | Water | 55.64 |
| A | Sodium Chloride | Sodium Chloride | 1.09 |
| | Glycerin | Glycerin | 2.19 |
| | Bentone Gel Abo V | Crambe Abyssinica Seed Oil, Stearalkoium Hectorite, Propylene Carbinate | 7.04 |
| | Imwittor 600 | Polyglyceryl-3 Polyricinoleate | 3.28 |
| | Jeescreen OMC | Ethylhexyl Methoxvcinnamate | 8.19 |
| B | Vitamin E Acetate USP | Tocopheryl Acetate | 0.11 |
| | Fancor Meadowfoam Seed Oil | Limnanthes Alba (Meadowfoam) Seed Oil | 3.28 |
| | Dub ININ | Isononyl Isononanoate | 4.91 |
| | CERAPHYL® 375 | Isostearvl Neopentanoate | 4.91 |
| C | Euxyl K701 | Phenoxyethanol, Benzoic Acid, Dehydroacetic Acid, Ethylhexylglycerin | 1.09 |

(continued)

| ϕ | Ingredients | INCI | % |
|---|---|---|---|
| | Gatuline Expression | Alcohol & Water & Acmella Oleracea Extract | 1.09 |
| | Vegetol Aloe ME200 Hvdro | Water & Propylene Glycol & Aloe Ferox Leaf Extract | 1.09 |
| | Vegetol Cp GR049 Hydro | Propylene Glycol & Cucumis Sativus Fruit Extract | 1.09 |
| D | Filler pigment | Inv. Ex 1-6 ; Comp. Ex. 7-10 (see Table 4) | 5.00 |
| | Total | | 100 |

The CC cream is made by combining the ingredients of components A and B separately, and heating each to 85°C. The warmed Component A is added to Component B and mixed using a Dispermat for 10 min. The mixture is cooled below 40°C and Component C is added. Phase D may be added to ~10g of formulation and mixed via Centrifugal mixer at 3,000 RPM for 1 min.

Table 4: Recipe of a generic pressed powder containing the Examples of the invention.

| Ingredient | Role | Supplier | Amount (g) |
|---|---|---|---|
| Yellow Iron Oxide | Pigment | Sun Chemical | 7.0 |
| Example from invention | Filler | Various | 11 |
| Trihydroxystearin | Binder | Elementis | 1.6 |

**Claims**

1.  A layered pigment system comprising a mineral substrate and a porous mineral shell, wherein the porous mineral shell comprises a calcium phosphate mineral.

2.  The layered pigment system of claim 1 wherein the mineral substrate is porous.

3.  The layered pigment system of claim 1, wherein the mineral substrate is a particle having a d50 from 2-100 $\mu$m.

4.  The layered pigment system of claim 1, wherein the mineral substrate is a particle having a d50 from 0.5-35 $\mu$m.

5.  The layered pigment system of claim 2, wherein the porous mineral substrate is crystalline, amorphous or a combination thereof.

6.  The layered pigment system of claim 5, wherein the porous mineral substrate is selected from the group consisting of silica, perlite, diatomaceous earth, kaolin, mica, kaolinite, sericite, clay, talc, diatomite, aluminum oxide, calcium carbonate, zeolite, and mixtures thereof.

7.  The layered pigment system of claim 6, wherein the porous mineral substrate is diatomaceous earth.

8.  The layered pigment system of claim 7, wherein is the calcium phosphate mineral is selected from the group consisting of calcium phosphate, monocalcium phosphate, monocalcium phosphate monohydrate, dicalcium phosphate, dicalcium phosphate dihydrate, dicalcium phosphate monohydrate, tricalcium phosphate, tetracalcium phosphate, octacalcium phosphate, dicalcium diphosphate, calcium triphosphate, calcium hydroxy phosphate, monetite, brushite, apatite, hydroxyapatite, and mixtures thereof.

9.  The layered pigment system of claim 8, wherein the calcium phosphate mineral is hydroxyapatite.

10. The layered pigment system of claim 1, which is blended or treated with one or more additives.

11. The layered pigment system of claim 10, wherein the additive is selected from the group consisting of methicone, dimethicone, trifluoropropyl dimethicone, lecithin, egg lecithin, vegetable lecithin, hydrogenated lecithingalatose arabinan sugar, starch, alginic acid, sodium alginate, potassium alginate, chitosan, magnesium myristate, aluminum myristate, zinc myristate, sodium glycerophosphate, alanine, arginine, asparagine, aspartic acid, sodium aspartate, cysteine, glutamine, glutamic acid, sodium glutamate, glycine, proline, histidine, isoleucine, leucine, lysine, methionine, phenylalanine, serine, threonine, tryptophan, tyrosine, valine, taurine, citruline, ornithine, theanine, dipeptides, tripeptides, polypeptides of more than three amino acids, proteins, enzymes, betaine, carnitine, carnosine, hydroxytryptophan, cysteine, hydroxyproline, N-acetyl cystine, S-adenosyl methionine, tyramine, $\gamma$-aminobutyric acid, serotonin, dopamine, 2-aminohepanoic acid, 2-aminoisobutyric acid, 3-aminoisobutyric acid, 2-aminopimelic acid, 2,4-diaminobutyric acid, desmosine, 2,2'-diaminopimelic acid, 2,3-diaminopropionic acid, N-ethylgllycine, selenomethionine, allo-isoleucine, N-methylglycine, N-methylisoleucine, 6-N-methyllysine, N-methylvaline, norvaline, norleucine, pyrrolysine, formylmethionine, $\beta$-alanine, $\delta$-aminolevulinic acid, 4-aminobenzoic acid, dehydroalanine, cystathionine, lanthionine, djenkolic acid, diaminpimelic acid, isovaline, lauroyl lysine, glutamate-cysteine-arginine peptides, sodium myristoyl sarcosinate, disodium steroyl glutamate, fatty acids, lipids, stearic acid, sodium stearate, oleic acid, sodium oleate, palmitic acid, sodium palmitate, myristic acid, elaidic acid, sodium elaidate, sodium myristate, lauric acid, sodium laurate, arachidic acid, sodium arachidate, erucic acid, sodium erucate, palmitoleic acid, sodium palmitoleate, linoleic acid, sodium linoleate, triethoxyoctyl silane, trimethoxyoctyl silane, triethoxydecyl silane, trimethoxydecyl silane, triethoxydodecyl silane, trimethoxydodecyl silane, triethoxytetradecyl silane, trimethoxytetradecyl silane triethoxyhexadecyl silane, trimethoxyhexadecyl silane, triethoxyoctadecyl silane, trimethoxyoctadecyl silane, PEG-8 triethoxysilane, jojoba wax, polyethylene wax, carnauba wax, unreacted fluorinated compounds, isopropyltitanium triisostearate, perfluoro alkyl phosphates, triethoxylcapryl silane, stearoyl glutamic acid, perfluoroocctyl triethoxysilane, silica, aloe, and mixtures and combinations thereof.

**12.** A cosmetic or personal care formulation comprising the layered pigment system of claim 1.

**13.** The cosmetic or personal care formulation of claim 12, wherein the cosmetic formulation is selected from the from the group consisting of foundations, pressed powders, loose powders, bronzers, concealers, liquid facial cosmetics, BB/CC creams, tinted moisturizers, liquid foundations, eye shadows, eye liners, lipsticks, lip glosses, blushes, rouges, facial powders, and nail polishes.

**14.** An ink or coating composition comprising the layered pigment system of claim 1, optionally wherein the ink or coating composition is selected from the group consisting of automotive coatings, protective clear coatings, interior architectural coatings, exterior architectural coatings, powder coatings, industrial coating, anti-corrosion coating, gravure inks, flexographic inks, paste inks, energy curing (UV or EB) inks.

**15.** A plastic material comprising the layered pigment system of claim 1, optionally wherein the plastic material is selected from polypropylene, polyethylene, polyester, polyurethane, polyacrylate, polyolefin, epoxy, polyamide, poly(vinyl chloride), and poly(vinylidene fluoride), as well as any acrylic, alkyd, fluoropolymers, and blends thereof.

**Patentansprüche**

**1.** Schichtpigmentsystem, umfassend ein mineralisches Substrat und eine poröse mineralische Schale, wobei die poröse mineralische Schale ein Calciumphosphatmineral umfasst.

**2.** Schichtpigmentsystem gemäß Anspruch 1, wobei das mineralische Substrat porös ist.

**3.** Schichtpigmentsystem gemäß Anspruch 1, wobei das mineralische Substrat ein Partikel mit einem d50 von 2-100 pm ist.

**4.** Schichtpigmentsystem gemäß Anspruch 1, wobei das mineralische Substrat ein Partikel mit einem d50 von 0,5-35 pm ist.

**5.** Schichtpigmentsystem gemäß Anspruch 2, wobei das poröse mineralische Substrat kristallin, amorph oder eine Kombination davon ist.

**6.** Schichtpigmentsystem gemäß Anspruch 5, wobei das poröse mineralische Substrat ausgewählt ist aus der Gruppe bestehend aus Siliciumdioxid, Perlit, Diatomeenerde, Kaolin, Glimmer, Kaolinit, Sericit, Ton, Talkum, Diatomit, Aluminiumoxid, Calciumcarbonat, Zeolith und Gemischen davon.

7. Schichtpigmentsystem gemäß Anspruch 6, wobei das poröse mineralische Substrat Diatomeenerde, ist.

8. Schichtpigmentsystem gemäß Anspruch 7, wobei das Calciumphosphatmineral ausgewählt ist aus der Gruppe bestehend aus Calciumphosphat, Monocalciumphosphat, Monocalciumphosphatmonohydrat, Dicalciumphosphat, Dicalciumphosphatdihydrat, Dicalciumphosphatmonohydrat, Tricalciumphosphat, Tetracalciumphosphat, Octacalciumphosphat, Dicalciumdiphosphat, Calciumtriphosphat, Calciumhydroxyphosphat, Monetit, Brushit, Apatit, Hydroxyapatit und Gemischen davon.

9. Schichtpigmentsystem gemäß Anspruch 8, wobei das Calciumphosphatmineral Hydroxyapatit ist.

10. Schichtpigmentsystem gemäß Anspruch 1, das mit einem oder mehreren Zusatzstoffen gemischt oder behandelt ist.

11. Schichtpigmentsystem gemäß Anspruch 10, wobei der Zusatzstoff ausgewählt ist aus der Gruppe bestehend aus Methicon, Dimethicon, Trifluorpropyldimethicon, Lecithin, Eilecithin, Pflanzenlecithin, hydriertem Lecithingalatosearabinanzucker, Stärke, Alginsäure, Natriumalginat, Kaliumalginat, Chitosan, Magnesiummyristat, Aluminiummyristat, Zinkmyristat, Natriumglycerophosphat, Alanin, Arginin, Asparagin, Asparaginsäure, Natriumaspartat, Cystein, Glutamin, Glutaminsäure, Natriumglutamat, Glycin, Prolin, Histidin, Isoleucin, Leucin, Lysin, Methionin, Phenylalanin, Serin, Threonin, Tryptophan, Tyrosin, Valin, Taurin, Citrulin, Ornithin, Theanin, Dipeptiden, Tripeptiden, Polypeptiden mit mehr als drei Aminosäuren, Proteinen, Enzymen, Betain, Carnitin, Carnosin, Hydroxytryptophan, Cystein, Hydroxyprolin, N-Acetylcystin, S-Adenosylmethionin, Tyramin, $\gamma$-Aminobuttersäure, Serotonin, Dopamin, 2-Aminohepansäure, 2-Aminoisobuttersäure, 3-Aminoisobuttersäure, 2-Aminopimelinsäure, 2,4-Diaminobuttersäure, Desmosin, 2,2'-Diaminopimelinsäure, 2,3-Diaminopropionsäure, N-Ethylglycin, Selenomethionin, Alloisoleucin, N-Methylglycin, N-Methylisoleucin, 6-N-Methyllysin, N-Methylvalin, Norvalin, Norleucin, Pyrrolysin, Formylmethionin, $\beta$-Alanin, $\delta$-Aminolevulinsäure, 4-Aminobenzoesäure, Dehydroalanin, Cystathionin, Lanthionin, Djenkolsäure, Diaminpimelinäure, Isovalin, Lauroyllysin, Glutamat-Cystein-Arginin-Peptiden, Natriummyristoylsarcosinat, Dinatriumsteroylglutamat, Fettsäuren, Lipiden, Stearinsäure, Natriumstearat, Ölsäure, Natriumoleat, Palmitinsäure, Natriumpalmitat, Myristinsäure, Elaidinsäure, Natriumelaidat, Natriummyristat, Laurinsäure, Natriumlaurat, Arachidinsäure, Natriumarachidat, Erucasäure, Natriumerucat, Palmitolsäure, Natriumpalmitoleat, Linolsäure, Natriumlinoleat, Triethoxyoctylsilan, Trimethoxyoctylsilan, Triethoxydecylsilan, Trimethoxydecylsilan, Triethoxydodecylsilan, Trimethoxydodecylsilan, Triethoxytetradecylsilan, Trimethoxytetradecylsilan, Triethoxyhexadecylsilan, Trimethoxyhexadecylsilan, Triethoxyoctadecylsilan, Trimethoxyoctadecylsilan, PEG-8-Triethoxysilan, Jojobawachs, Polyethylenwachs, Carnaubawachs, unreagierte fluorierte Verbindungen, Isopropyltitantriisostearat, Perfluoralkylphosphate, Triethoxycaprylsilan, Stearoylglutaminsäure, Perfluoroctyltriethoxysilan, Siliciumdioxid, Aloe und Gemischen und Kombinationen davon.

12. Kosmetische oder Körperpflegeformulierung, umfassend das Schichtpigmentsystem gemäß Anspruch 1.

13. Kosmetische oder Körperpflegeformulierung gemäß Anspruch 12, wobei die kosmetische Formulierung ausgewählt ist aus der Gruppe bestehend aus Grundlagen, gepressten Pulvern, losen Pulvern, Bräunungsmitteln, Abdeckern, flüssigen Gesichtskosmetika, BB/CC-Cremen, getönten Feuchtigkeitsmitteln, flüssigen Grundlagen, Lidschatten, Eyelinern, Lippenstiften, Lipglossen, Blushes, Rouges, Gesichtspulvern und Nagellacken.

14. Tinte oder Beschichtungszusammensetzung, umfassend das Schichtpigmentsystem gemäß Anspruch 1, wobei die Tinte oder Beschichtungszusammensetzung gegebenenfalls ausgewählt ist aus der Gruppe bestehend aus Automobilbeschichtungen, klaren Schutzbeschichtungen, Innenarchitekturbeschichtungen, Außenarchitekturbeschichtungen, Pulverbeschichtungen, industrieller Beschichtung, Antikorrosionsbeschichtung, Tiefdrucktinten, Flexodrucktinten, pastösen Tinten, energiehärtenden (UV oder EB) Tinten.

15. Kunststoffmaterial, umfassend das Schichtpigmentsystem gemäß Anspruch 1, wobei das Kunststoffmaterial gegebenenfalls ausgewählt ist aus Polypropylen, Polyethylen, Polyester, Polyurethan, Polyacrylat, Polyolefin, Epoxy, Polyamid, Poly(vinylchlorid) und Poly(vinylidenfluorid) sowie einem Acryl, Alkyd, Fluorpolymeren und Gemischen davon.

## Revendications

1. Système de pigment stratifié comprenant un substrat minéral et une enveloppe minérale poreuse, l'enveloppe minérale poreuse comprenant un minéral de phosphate de calcium.

2. Système de pigment stratifié selon la revendication 1, le substrat minéral étant poreux.

3. Système de pigment stratifié selon la revendication 1, le substrat minéral étant une particule ayant un d50 de 2 à 100 pm.

4. Système de pigment stratifié selon la revendication 1, le substrat minéral étant une particule ayant un d50 de 0,5 à 35 pm.

5. Système de pigment stratifié selon la revendication 2, le substrat minéral poreux étant cristallin, amorphe ou une combinaison correspondante.

6. Système de pigment stratifié selon la revendication 5, le substrat minéral poreux étant choisi dans le groupe constitué par une silice, une perlite, une terre à diatomées, un kaolin, un mica, une kaolinite, une séricite, une argile, un talc, une diatomite, l'oxyde d'aluminium, le carbonate de calcium, une zéolithe et des mélanges correspondants.

7. Système de pigment stratifié selon la revendication 6, le substrat minéral poreux étant une terre à diatomées.

8. Système de pigment stratifié selon la revendication 7, le minéral de phosphate de calcium étant choisi dans le groupe constitué par le phosphate de calcium, le phosphate monocalcique, le monohydrate de phosphate monocalcique, le phosphate dicalcique, le dihydrate de phosphate dicalcique, le monohydrate de phosphate dicalcique, le phosphate tricalcique, le phosphate tétracalcique, le phosphate octacalcique, le diphosphate dicalcique, le triphosphate de calcium, l'hydroxyphosphate de calcium, la monétite, la brushite, l'apatite, l'hydroxyapatite et des mélanges correspondants.

9. Système de pigment stratifié selon la revendication 8, le minéral de phosphate de calcium étant l'hydroxyapatite.

10. Système de pigment stratifié selon la revendication 1, qui est mélangé ou traité par un ou plusieurs additifs.

11. Système de pigment stratifié selon la revendication 10, l'additif étant choisi dans le groupe constitué par méthicone, diméthicone, trifluoropropyldiméthicone, lécithine, lécithine d'œuf, lécithine végétale, lécithine hydrogénée, galactose, sucre arabinane, amidon, acide alginique, alginate de sodium, alginate de potassium, chitosane, myristate de magnésium, myristate d'aluminium, myristate de zinc, glycérophosphate de sodium, alanine, arginine, asparagine, acide aspartique, aspartate de sodium, cystéine, glutamine, acide glutamique, glutamate de sodium, glycine, proline, histidine, isoleucine, leucine, lysine, méthionine, phénylalanine, sérine, thréonine, tryptophane, tyrosine, valine, taurine, citruline, ornithine, théanine, dipeptides, tripeptides, polypeptides de plus de trois acides aminés, protéines, enzymes, bétaïne, carnitine, carnosine, hydroxytryptophane, cystéine, hydroxyproline, N-acétylcystine, S-adénosylméthionine, tyramine, acide $\gamma$-aminobutyrique, sérotonine, dopamine, acide 2-aminohepanoïque, acide 2-aminoisobutyrique, acide 3-aminoisobutyrique, acide 2-aminopimélique, acide 2,4-diaminobutyrique, desmosine, acide 2,2'-diaminopimélique, acide 2,3-diaminopropionique, N-éthylglycine, sélénométhionine, allo-isoleucine, N-méthylglycine, N-méthylisoleucine, 6-N-méthyllysine, N-méthylvaline, norvaline, norleucine, pyrrolysine, formylméthionine, $\beta$-alanine, acide $\delta$-aminolévulinique, acide 4-aminobenzoïque, déhydroalanine, cystathionine, lanthionine, acide djenkolique, acide diaminpimélique, isovaline, lauroyllysine, peptides glutamate-cystéine-arginine, myristoylsarcosinate de sodium, stéroylglutamate disodique, acides gras, lipides, acide stéarique, stéarate de sodium, acide oléique, oléate de sodium, acide palmitique, palmitate de sodium, acide myristique, acide élaidique, élaïdate de sodium, myristate de sodium, acide laurique, laurate de sodium, acide arachidique, arachidate de sodium, acide érucique, éruciate de sodium, acide palmitoléique, palmitoléate de sodium, acide linoléique, linoléate de sodium, triéthoxyoctylsilane, triméthoxyoctylsilane, triéthoxydécylsilane, triméthoxydécylsilane, triéthoxydodécylsilane, triméthoxytétradécylsilane, triméthoxytétradécylsilane triéthoxyhexadécylsilane, triméthoxyhexadécylsilane, triéthoxyoctadécylsilane, triméthoxyoctadécylsilane, PEG-8 triéthoxysilane, cire de jojoba, cire de polyéthylène, cire de carnauba, composés fluorés n'ayant pas réagi, triisostéarate d'isopropyltitane, phosphates d'alkyle perfluoré, triéthoxylcaprylsilane, acide stéaroylglutamique, perfluoroocctyltriéthoxysilane, silice, aloès et des mélanges et des combinaisons correspondantes.

12. Formulation cosmétique ou de soin personnel comprenant le système de pigment stratifié selon la revendication 1.

13. Formulation cosmétique ou de soin personnel selon la revendication 12, la formulation cosmétique étant choisie dans le groupe constitué par des fonds de teint, des poudres pressées, des poudres libres, des agents bronzants, des anticernes, des produits cosmétiques liquides pour le visage, des crèmes BB/CC, des agents hydratants teintés, des fonds de teint liquides, des ombres à paupières, des crayons pour les yeux, des rouges à lèvres, des brillants à lèvres, des fards à joues, des poudres pour le visage et des vernis à ongles.

14. Composition d'encre ou de revêtement comprenant le système de pigment stratifié selon la revendication 1, éventuellement, la composition d'encre ou de revêtement étant choisie dans le groupe constitué par des revêtements pour automobiles, des revêtements transparents protecteurs, des revêtements d'architecture d'intérieur, des revêtements d'architecture d'extérieur, des revêtements en poudre, un revêtement industriel, un revêtement anti-corrosion, des encres de gravure, des encres flexographiques, des encres en pâte, des encres durcissant par énergie (UV ou EB).

15. Matière plastique comprenant le système de pigment stratifié selon la revendication 1, éventuellement, la matière plastique étant choisie parmi un polypropylène, un polyéthylène, un polyester, un polyuréthane, un polyacrylate, une polyoléfine, un époxy, un polyamide, un poly(chlorure de vinyle) et un polyfluorène de vinylidène, ainsi qu'un quelconque acrylique, un quelconque alkyde, de quelconques fluoropolymères et des mélanges correspondants.

**Figure 1**: SEM image of Comparative Example 7

**Figure 2**: SEM imaging of Example 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017127362 A1

**Non-patent literature cited in the description**

- **FARRAH et al.** *Applied and Environmental Microbiology,* 1991, vol. 57 (9), 2502-2506

- **DONG et al.** *Powder Technology,* 2020, vol. 366, 537-545